# FASCICULE DE BREVET EUROPEEN

(11) **EP 1 689 385 B1**
(45) Date de publication et mention de la délivrance du brevet: **11.04.2007**
(21) Numéro de dépôt: 04805558.6
(22) Date de dépôt: 25.11.2004
(51) Int. Cl.: A61K 31/336, A61P 25/16, A01N 43/20, C07D 303/16

(54) **SUBSTANCE NEUROACTIVE ET UTILISATIONS D'UNE TELLE SUBSTANCE**
NEUROAKTIVE SUBSTANZ UND VERWENDUNGEN ENER SOLCHEN SUBSTANZ
NEUROACTIVE SUBSTANCE AND USES OF ONE SUCH SUBSTANCE

(30) Priorité: 02.12.2003 FR 0314167
(43) Date de publication de la demande: 16.08.2006
(73) Titulaire: UNIVERSITE DE NANTES, 44322 Nantes (FR); UNIVERSITE D'ANGERS, 49035 Angers (FR)
(72) Inventeur: PETIT, Karina-Ethel, F-44700 Orvault (FR); BIARD, Jean-François, F-44300 Nantes (FR); LAPIED, Bruno, F-44000 Nantes (FR); GROLLEAU, Françoise, 49100 Angers (FR); HAMON, Alain, F-49100 Angers (FR)
(74) Mandataire: Larcher, Dominique
(86) Numéro de dépôt international: PCT/FR2004/003030
(87) Numéro de publication internationale: WO 2005/056000

(56) Documents cités:
- MORRIS L A ET AL: "A bioactive secosterol with an unusual A- and B-ring oxygenation pattern isolated from an Indonesian soft coral Lobophytum sp." JOURNAL OF NATURAL PRODUCTS 1998 UNITED STATES, vol. 61, no. 4, 1998, pages 538-541, XP001193731 ISSN: 0163-3864
- GREEN D ET AL: "Secondary metabolites of the yellow and gray morphs of the soft coral Parerythropodium fulvum fulvum: Comparative aspects" JOURNAL OF NATURAL PRODUCTS (LLOYDIA) 1992 UNITED STATES, vol. 55, no. 9, 1992, pages 1186-1196, XP008031532 ISSN: 0163-3864
- NAZ S ET AL: "New antiproliferative epoxysecosterols from Pseudopterogorgia americana" TETRAHEDRON LETTERS 05 AUG 2000 UNITED KINGDOM, vol. 41, no. 32, 5 août 2000 (2000-08-05), pages 6035-6040, XP002284697 ISSN: 0040-4039

## Description

L'invention concerne principalement le domaine de la biochimie.

Plus précisément, l'invention concerne le domaine des substances neuroactives.

Les cellules sont délimitées par des membranes qui présentent des perméabilités sélectives pour plusieurs ions, essentiellement les ions Na⁺, K⁺, Ca²⁺ et Cl⁻.

Ces perméabilités sélectives sont assurées par des canaux ioniques qui sont constitués par des protéines transmembranaires formant des pores au travers des membranes cellulaires.

Ces pores autorisent les flux transmembranaires passifs d'ions et jouent ainsi des rôles prépondérants dans de nombreuses fonctions cellulaires telles que l'excitation, la transmission synaptique, la sécrétion ou la contraction.

Les flux ioniques au travers de la membrane sont soumis à une force électrochimique déterminée à la fois par une répartition asymétrique de chacun des ions de part et d'autre de la membrane, c'est-à-dire un gradient de concentration, et par un champ électrique, suivant l'équation de Nernst.

Toutes espèces ioniques considérées, le système évolue vers un état d'équilibre qui définit le potentiel de la cellule au repos. Selon le type cellulaire, ce potentiel varie entre -40 mV et -90mV.

Les canaux ioniques ont donc un rôle primordial dans la fonction cellulaire et beaucoup d'applications sont attendues pour des produits actifs sur ceux-ci.

De telles substances neuroactives agissant sur les canaux ioniques présentent notamment un intérêt pour la recherche fondamentale, puisque employés comme agents pharmacologiques, ils permettent de mieux connaître les mécanismes des fonctions nerveuses.

Parallèlement, on sait qu'un certain nombre de maladies ou syndromes sont liés aux troubles de l'excitabilité neuronale. De telles substances neuroactives agissant sur les canaux ioniques peuvent donc aussi aider au développement de nouveaux médicaments.

De nombreux domaines liés à un dérèglement du système nerveux central sont ainsi à l'heure actuelle explorés : les douleurs neuropathiques (spontanées ou associées aux opérations, cancers, zonas etc.), les maladies neurodégénératives (maladie d'Alzheimer, maladie de Parkinson), les troubles psychiques (schizophrénie) ou neurologiques (épilepsie).

Il existe donc un besoin pour de nouvelles substances neuroactives et notamment pour celles susceptibles d'agir sur les canaux ioniques.

Depuis une cinquantaine d'années, la recherche de métabolites bioactifs s'est tournée vers le monde marin. En effet, de par leur incroyable biodiversité, les espèces océaniques constituent un gigantesque réservoir de substances naturelles.

Par exemple, en ce qui concerne les substances neuro-actives, l'oméga-conotoxine a été isolée à partir d'un mollusque (*Conus magus*). Cette substance, qui bloque certains canaux calciques, présente une efficacité 100 à 1000 fois supérieure à celle de la morphine. Elle est maintenant utilisée pour la réalisation d'un médicament destiné à lutter contre la douleur : le ziconotide (marque déposée).

Un objectif de la présente invention est donc de proposer une nouvelle substance neuroactive agissant sur les canaux ioniques membranaires.

En particulier, un objectif de la présente invention est de proposer une telle substance susceptible d'agir sur un type de canal calcique.

Encore un autre objectif de la présente invention est de proposer une telle substance présentant une potentialité pour la réalisation de médicaments destinés à lutter contre les troubles de l'excitabilité neuronale (canalopathies).

Encore un autre objectif de la présente invention est de présenter une telle substance neuroactive qui pourrait, le cas échéant, être utilisée comme insecticide, ou antagoniste, chez l'homme, des effets toxiques de certains insecticides.

Ces différents objectifs sont atteints grâce à l'invention qui concerne une substance neuroactive caractérisée en ce qu'elle répond à la formule (O) 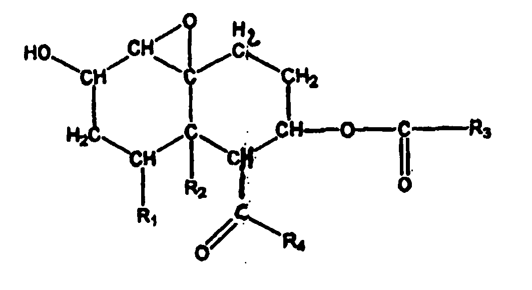 dans laquelle R1, R2, R3 et R4 sont identiques ou différents et sont des radicaux méthyle ou éthyle.

Préférentiellement, ladite substance répond à la formule (I) 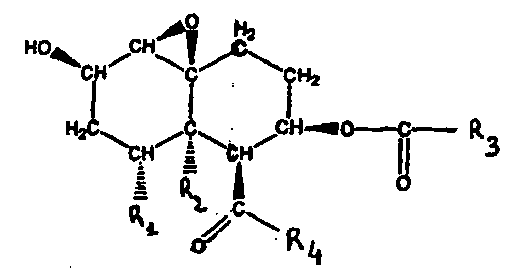 qui présente une stéréochimie particulière par rapport à la formule (O)

De façon préférée entre toutes, cette substance est constituée par le 6S-acétyl-4R,5R-diméthyl-1R(10S)-époxy-2R-hydroxy-7R-acétoxydécahydronaphtalène de formule (II)

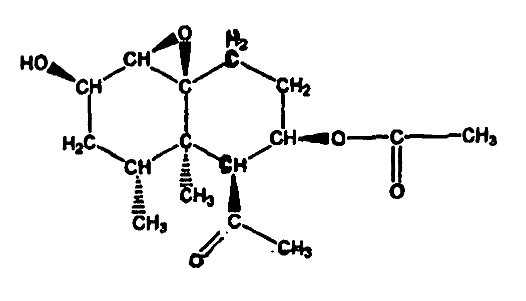

Une telle substance répond à la formule (I) indiquée ci-dessus avec R1, R2, R3 et R4 constitué chacun par un radical méthyle.

Cette substance a été isolée par les inventeurs chez un corail, à savoir le cnidaire *Rhytisma fulvum.*

L'espèce *Rhytisma fulvum* appartient à la famille des *Alcyoniidae* à l'ordre des *Alcyonacea,* à la sous-classe *Octocorallia.* à la classe *Anthozoa,* et à l'embranchement *Cnidaria.* Cette espèce de cnidaire a été décrite par Forskal en 1775 et par Alderslade en 2000 (Zool.Med.Leiden, 2000, 74(16) : 237-249).

*Rhytisma fulvum* est un corail qui présente une large distribution bathymétrique puisqu'il s'étend de -3m à -40m. Sa distribution géographique le localise dans les mers tropicales sur des sites ponctuels mais d'une manière particulièrement abondante. On le trouve notamment en Mer Rouge, à Zanzibar, à Madagascar, aux Iles Paternoster (Indonésie), en Papouasie, en Nouvelle-Guinée et le long de la grande barrière de corail australienne.

De nombreux métabolites ont déjà été isolés de *Rhytisma fulvum* notamment par Bowden *et al.* Tetrahedron Lett., 1980, 21 (32) : 3105-3108, par Green *et al.* J.Nat.Prod., 1992, 55 (9) : 1186-1196 et Wessels *et al.* J.Nat.Prod., 2001, 64 (3) : 370-372. Aucune activité pharmacologique n'a été associée à ceux-ci.

Tous ces métabolites appartiennent à la classe chimique des terpènes. A la connaissance de la Demanderesse le métabolite de formule (II) n'a jamais été décrit.

Tel qu'il sera exposé ci-après plus en détails, les inventeurs ont prouvé que cette substance de formule (II) présentait une neuroactivité chez la blatte. Plus précisément, les chercheurs ont prouvé que cette substance de formule (II) était un activateur spécifique des canaux calciques transitoires à bas seuil d'activation, ce qui en fait, à leur connaissance le premier activateur membranaire de ce type de canaux calcium.

Cette substance (II) pourra donc être utilisée en recherche fondamentale à titre de réactif pharmacologique notamment dans le cadre de travaux impliquant les canaux ioniques membranaires.

Pour l'instant, les chercheurs n'ont pas encore déterminé si cette neuroactivité était spécifique aux insectes ou existait aussi chez le mammifère, voire chez l'être humain.

Si la neuroactivité de cette substance se révèle ultérieurement spécifique aux insectes, cette substance sera susceptible d'être utilisée pour la réalisation d'insecticides. Dans ce cadre, cette substance pourra être utilisée seule ou en combinaison avec au moins un autre insecticide tels que notamment ceux induisant une neuroactivité contraire à celle du composé selon l'invention.

Si au contraire, la neuroactivité de cette substance est également observée chez l'être humain, cette substance pourra être utilisée pour
- la fabrication de médicaments activateurs de neurones dopaminergiques par exemple pouvant être utilisé notamment pour lutter contre la maladie de Parkinson qui est caractérisée entre autre par une diminution de la fréquence des potentiels d'action de ce type de neurones ;
- la fabrication de médicaments destinés à lutter contre la diminution de la fréquence des potentiels d'action des neurones à activité pacemaker. Cette fabrication pourra être étendue à une utilisation dans la lutte contre les troubles cardiaques.

On notera que la substance de formule (II) isolée par les inventeurs chez *Rhytisma fulvum* pourra tout à fait être synthétisée par voie chimique. Dans ce cadre, il pourra être obtenu des substances de formule (I) dans lesquelles les radicaux R1, R2, R3, R4 seront constitués par des radicaux méthyle et/ou éthyle. Les inventeurs estiment que les substances de formule (O) ou de formule (I), proches de la substance de formule (II), sont susceptibles de présenter eux aussi une neuroactivité et donc d'être utilisées pour les mêmes applications que celles indiquées ci-dessus.

L'invention, ainsi que les différents avantages qu'elle présente seront plus facilement compris grâce à la description qui va suivre des travaux menés par les inventeurs démontrant la neuroactivité chez la blatte (*Periplaneta americana*) de la substance de formule (II). Cette description donnée en référence aux figures dans lesquelles :
- les figures 1 et 2 concernent les trois types de courants calciques ;
- les figures 3, 4 et 5 concernent l'effet de la substance de formule (II) sur l'activité électrique spontanée des neurones DUM de la blatte ;
- la figure 6 concerne l'influence de la substance de formule (II) sur des potentiels d'action déclenchés sur des neurones DUM de la blatte ;
- la figure 7 concerne l'effet de la substance de formule (II) sur le courant calcique HVA lors d'une impulsion dépolarisante ;
- la figure 8 concerne l'effet de la substance de formule (II) sur l'amplitude du courant calcique LVA global en fonction du temps ;
- la figure 9 concerne l'effet de la substance de formule (II) sur l'amplitude du courant calcique LVA global en réponse à une impulsion dépolarisante ;
- la figure 10 concerne l'effet de la substance de formule (II) en présence de chlorure de nickel sur l'amplitude du courant calcique LVA m et HVA ;
- la figure 11 concerne l'effet de la substance de formule (II) en présence de chlorure de nickel sur l'amplitude du courant calcique LVAm enregistré lors d'une impulsion dépolarisante ;

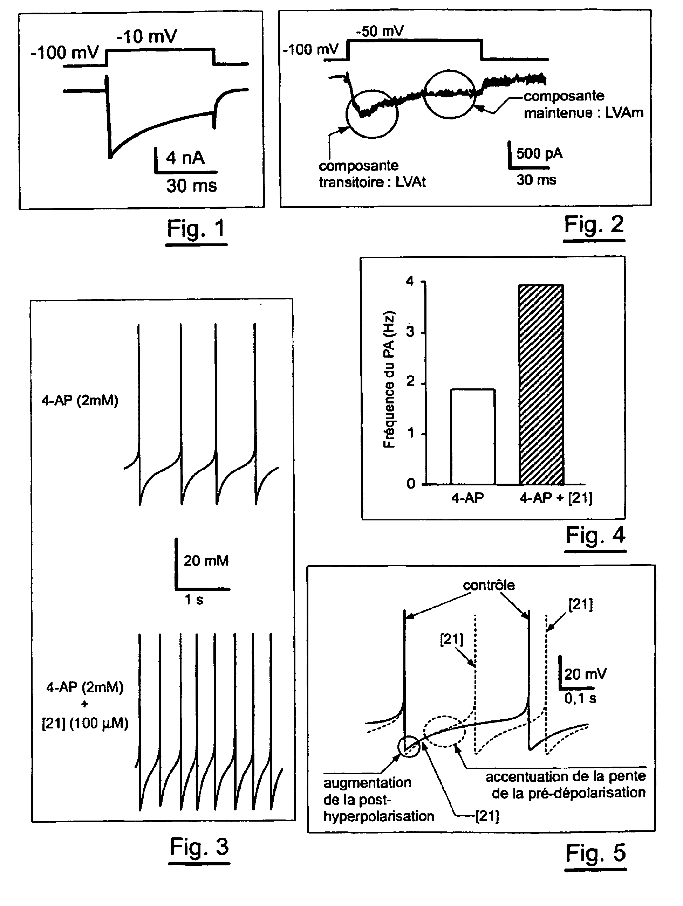

- les figures 3 à 21 résume les effets de la substance de formule (II) sur l'amplitude des différents courant calciques.

2625 g de corail *Rhytisma fulvum* pêché au large de Djibouti ont été utilisés pour obtenir un extrait éthanolique de 33,5 g (rendement 1,28%).

La substance de formule (II) a été extraite par un procédé classique de chromatographie selon le mode opératoire décrit ci-après.

L'extrait éthanolique de *Rhytisma fulvum* a été soumis à une Chromatographie Liquide sous Vide (CLV) sur silice. L'élution a été effectuée avec un mélange de dichlorométhane (CH₂Cl₂) et de méthanol (MeOH).

Une Chromatographie Liquide sous Vide avec une phase stationnaire apolaire C₁₈ a ensuite été effectuée sur la fraction CH₂Cl₂/MeOH 98 :2 obtenue à l'étape précédente avec un éluant constitué par un mélange de méthanol (MeOH) et d'eau.

Une Chromatographie Liquide sous Vide sur silice a ensuite été effectuée sur la fraction MeOH/H₂O 50 :50 obtenue à l'étape précédente avec un éluant constitué d'hexane et d'acétone.

Une Chromatographie Liquide Haute Performance sur gel de silice (Nucléosil® 5 µm Si) a ensuite été effectuée sur la fraction hexane/acétone 60/40 obtenue à l'étape précédente avec un éluant constitué de dichlorométhane et d'éthanol (EtOH)

La fraction présentant un temps de rétention compris entre 7 mn 30 et 9 mn a ensuite été ensuite soumise à une Chromatographie Liquide Haute Performance sur gel de silice (Nucléosil® 5 µm Si) en utilisant un éluant constitué de dichlorométhane et d'éthanol (EtOH) ce qui a permis l'obtention de deux fractions dont l'une présentant un temps de rétention compris entre 25 à 34 minutes. Cette fraction a été analysée et a permis de conclure qu'il s'agissait d'un composé de formule (II).

Lors de ces expériences, le matériel chromatographique utilisé pour la Chromatographie Liquide sous Vide présentait les caractéristiques suivantes :
- adsorbant : silice 60 Å, granulométrie 35-70 µm, *Chromagel SDS ;* C₁₈, 60 Å, granulométrie 60 µm, *Macherey-Nagel*
- colonne de verre avec de la laine de verre comme filtre.

Le matériel chromatographique utilisé pour la Chromatographie Liquide à Haute Performance présentait quant à lui les caractéristiques suivantes :
- manomètre : 806 module manométrique, *Gilson*
- pompe à solvants : 305 pump, *Gilson*
- injecteur : vanne Rheodyne, boucle 100 µL
- détecteurs : 115 UV et 132 RI, *Gilson*
- imprimante : SE120, *BBC Guerz Metrawatt*
- colonnes analytiques : 250 x 4.6 mm, Nucleosil 5 µm Si
- colonne préparative : 250 x 22 mm, Rsil 10 µm
- seringue : 100 µL ou 500 µL, *Hamilton.*

On notera que la substance de formule (II) a été obtenue avec un rendement de 1,05% par rapport à l'extrait éthanolique de départ, c'est-à-dire, 0,0013% en poids par rapport à la matière première.

La neuroactivité de la substance de formule (I) a ensuite été testée par injection sur des larves de diptères. Dans ce cadre, les inventeurs ont utilisé des larves de Cyloraphes de l'espèce *Phormia terrae novae* au stade larvaire III, juste avant le stade imago permettant la métamorphose en pupe ou en nymphe. Ce type de larves est disponible dans les magasins de pêche et se conserve dans du son à 4°C pendant 10 jours maximum.

L'injection du produit est réalisée à l'aide d'une micro-seringue de précision équipée d'une aiguille hypodermique biseautée. L'aiguille est insérée au niveau du dernier segment abdominal de l'animal face dorsale. L'injection est considérée comme réussie si la larve est toujours mobile au bout de l'aiguille. Dans ce type de test, une contraction ou une relaxation d'un minimum de 5 secondes est définie comme une réponse positive, l'intensité et la durée étant cependant dose-dépendante tandis que l'absence de tout symptôme sur 10 min constitue une réponse négative.

La substance (II) a été testée en injectant une dose unique de 350 µg de produit par larve (poids moyen des larves 70 mg), au moyen d'une injection de 7 µl d'une solution à 50 mg/ml du produit dans le mélange eau : DMSO 85-15 v/v.

Cette concentration a ensuite été diluée de moitié jusqu'à ce qu'aucune activité des larves soit détectée.

Ces tests ont permis d'établir que la concentration minimale active sur les larves de diptères de la substance de formule (II) est de 3,1 µg par µL et par 10 mg de larves.

Le poids moléculaire de la substance de formule (II) étant de 296 g par mole, la CMA de celle-ci est donc d'environ 1 mM / mg de larves.

Les inventeurs ont ensuite étudié les effets possibles de la substance de formule (II) sur les courants ioniques membranaires neuronaux.

Dans un premier temps, les canaux sodiques et potassiques neuronaux ont été étudiés. Ces canaux sont impliqués dans la genèse des potentiels d'action.

Cette étude a été menée sur des axones géants de blattes (*Periplaneta americana*)*.* L'axone géant de ces blattes a été isolé dans un connectif d'une chaîne nerveuse abdominale ventrale.

Lors de l'expérimentation, l'axone est baigné par du liquide physiologique. L'expérimentation se fait en potentiel imposé et en courant imposé en absence et en présence de la substance de formule (II).

Cette expérimentation n'a permis de constater aucun changement sur le potentiel d'action. La substance de formule (II) n'a donc pas d'activité sur les canaux sodiques et potassiques axonaux de blatte.

Une seconde expérimentation a été menée sur les neurones dorsaux impairs et médians (neurones DUM pour Dorsal Unpaired Median Neurons) du système nerveux central des blattes (*Periplaneta americana*). Ces neurones sécrètent notamment une amine biogène, l'octopamine, un neuromédiateur dont la structure chimique est proche de la dopamine des vertébrés. Ils constituent un bon modèle d'étude car ils présentent des propriétés électrophysiologiques proches de celles des neurones dopaminergiques des vertébrés.

Ces neurones DUM sont isolés à partir de la ligne médio-dorsale du dernier ganglion abdominal terminal de la chaîne nerveuse des blattes mâles adultes, par digestion enzymatique et dissociation mécanique, selon la technique décrite par Lapied *et al.* (J. Exp. Biol., 1989, 144 : 535-549). Les neurones ainsi isolés sont maintenus en culture à 29°C pendant 24h avant expérimentation. La technique du patch-clamp en configuration cellule entière est utilisée pour mesurer l'activité électrique ainsi que les courants calciques (conditions de potentiel et/ou de courant imposé) selon la méthode décrite par Grolleau et Lapied (J.Neurophysiol. 1995, 73 : 160-171).

Une telle expérimentation a eu pour objectif de déterminer sur quel type de canal calcique la substance de formule (II) agissait.

Il existe en effet deux grands groupes de canaux calciques, à savoir les canaux calciques à haut seuil d'activation (HVA pour High Voltage Activated calcium channel - voir figure 1) et les canaux calciques à bas seuil d'activation (LVA pour Low Voltage Activated calcium channel - voir figure 2). Parmi les canaux calciques à bas seuil d'activation, on distingue les canaux calciques LVA transitoires (LVAt) et les canaux calciques LVA maintenus (LVAm).

Les courants calciques HVA s'activent pour des potentiels proches de -30mV.

Les courants calciques LVA s'activent pour des potentiels plus négatifs compris entre -70 et -50 mV.

Le courant calcique LVA transitoire est caractérisé par un seuil d'activation à -70 mV. L'inactivation est dépendante du potentiel et indépendante de l'influx de Ca²⁺. Ce courant qui est sensible au chlorure de nickel (100 µM) est impliqué dans la phase initiale de pré-dépolarisation. Il correspond au courant de type T classique des vertébrés.

Le courant calcique LVA maintenu se distingue par un seuil d'activation à -60 mV, une inactivation dépendante du potentiel et de la concentration intracellulaire de Ca²⁺. Il est insensible au chlorure de nickel (100 µM) et est impliqué dans la phase terminale de la pré-dépolarisation.

Une première série d'expériences sur les neurones DUM a permis de voir un effet de la substance de formule (II) sur l'activité électrique « pacemaker ». Les expériences ont été réalisées en présence de 4-aminopyridine (4-AP, 2 mM) afin de bloquer le courant potassium de type A, lequel intervient normalement pour maintenir une fréquence basse de décharge des potentiels d'action (Grolleau et Lapied, 1995). En absence de 4-AP, l'activation de ce courant est susceptible de masquer une variation éventuelle de la fréquence de décharge des potentiels d'action.

Comme le montre la figure 3 ci-après, la fréquence des potentiels d'action est affectée, mais l'amplitude reste inchangée. En présence de la substance (II) à 100 µM, la fréquence est augmentée d'un facteur 2,1 qui est statistiquement significatif comme le montre la figure 4. La phase de post-hyperpolarisation est également augmentée comme le montre la figue 5. Enfin, la pente de la pré-dépolarisation est plus élevée, ce qui a pour conséquence un déclenchement plus précoce du potentiel d'action, sans changement dans sa durée, ni dans son amplitude.

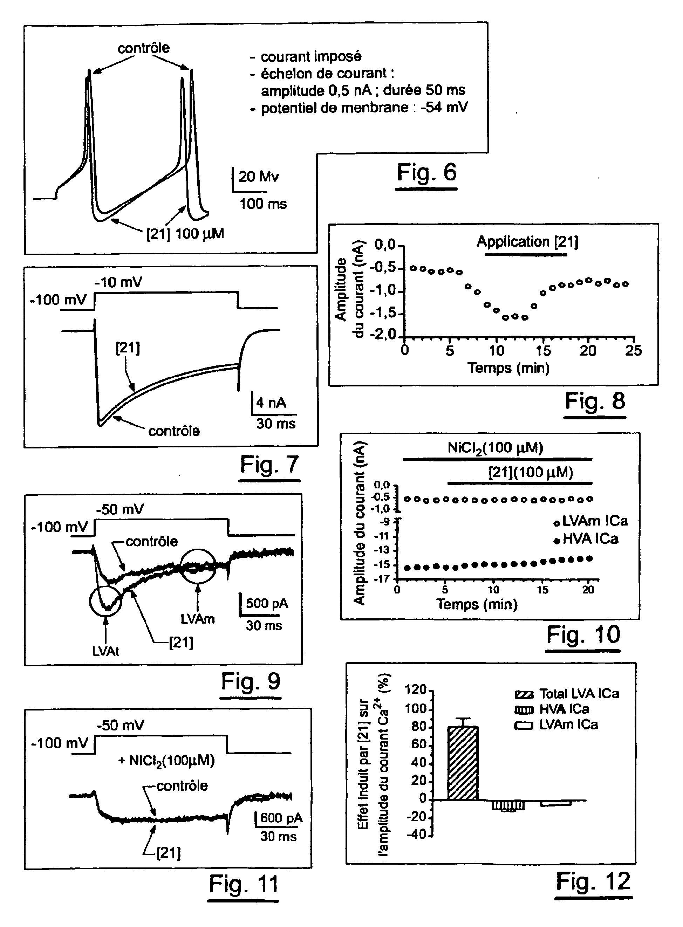

Des expériences ont également été réalisées sur une activité électrique déclenchée par injection d'un échelon de courant dépolarisant contrôlé en amplitude et en durée (figure 6). Les résultats obtenus dans ces conditions sont en accord avec les expériences réalisées sur l'activité spontanée. Une augmentation à la fois de la phase de post-hyperpolarisation et de la pente de la pré-dépolarisation est observée. Ceci se traduit par une augmentation de la fréquence de décharge, le seuil de déclenchement du potentiel d'action étant atteint plus tôt. Aucune modification dans l'amplitude ou la durée du potentiel d'action n'a été observé.

L'ensemble de ces observations tend à exclure un effet direct de la subsance de formule (II) sur les canaux sodiques et potassiques puisque respectivement ni l'amplitude du potentiel d'action ni sa durée ne sont affectées par sa présence. Ces observations confirment donc les résultats obtenus précédemment sur l'axone géant de blatte. Par contre, la subsance de formule (II) augmente la fréquence de décharge des potentiels d'actions. Or, ce sont principalement les courants calciques de type LVA, transitoire et maintenu, qui interviennent dans la phase de pré-dépolarisation.

Les deux groupes de courants calciques (*i.e.*, HVA et LVA) sont suffisamment différents pour que l'on puisse étudier séparément l'action de composés neuroactifs sur chacun d'eux. Grâce à l'utilisation d'un protocole expérimental permettant d'appliquer deux impulsions dépolarisantes d'amplitudes différentes, il est possible d'enregistrer uniquement le courant HVA (test à -10 mV à partir d'un potentiel de référence de -100 mV) ou le courant LVA global (test à -50 mV à partir d'un potentiel de référence de -100 mV). Dans le dernier cas, il est également possible d'enregistrer uniquement le courant LVAm en travaillant en présence de 100 µM de chlorure de nickel. Dans ces conditions, le courant LVAt s'obtient par « soustraction » du courant LVA global avec le LVAm. Dans tous les cas, les enregistrements sont obtenus en présence d'inhibiteurs spécifiques des courants sodiques et potassiques (100 nM de tétrodotoxine, 100 mM de TEA-CI et 5 mM de 4-AP, respectivement).

Lorsqu'une impulsion dépolarisante de +90 mV est appliquée au potentiel de référence de -100 mV, seul le courant calcique HVA est activé. Or, l'amplitude de ce courant est seulement réduite de 9% (n = 7) par rapport au contrôle en présence de la substance de formule (II). Cette variation n'est pas significative et permet de conclure que le produit agit très peu au niveau du courant calcique de type HVA (figure 7).

Lorsque la substance de formule (II) est appliquée sur les neurones DUM, l'amplitude du pic du courant calcique LVA global, enregistrée en réponse à une impulsion dépolarisante de +50 mV à partir d'un potentiel de référence de -100mV, augmente immédiatement (+81% ± 24 ; n = 7 ; figure 8). Par contre, il apparaît clairement que la composante maintenue mesurée en fin de l'impulsion dépolarisante n'est pas affectée (figure 9). Ces deux expériences orientent donc vers l'hypothèse que la substance de formule (II) affecte sélectivement un des deux types de canaux calciques LVA. Cette hypothèse a été vérifiée en réitérant l'expérience, mais cette fois en présence de 100 µM de chlorure de nickel connu pour bloquer sélectivement le courant calcique LVAt.

Les inventeurs ont constaté que ni l'amplitude du courant LVAm (figures 10 et 11), ni celle du HVA ne sont modifiées (figure 10). Cela confirme donc que la substance de formule (II) agit spécifiquement au niveau du LVAt.

La figure 12 résume les effets de la substance de formule (II) sur l'amplitude des différents courant calciques

La substance de formule (II) augmente donc la fréquence de décharge des potentiels d'action en potentialisant le canal calcique de type LVAt. Il y a donc entrée de calcium, donc accentuation de la pente de pré-dépolarisation qui permet d'atteindre plus rapidement le seuil de déclenchement du potentiel d'action.

L'analogie entre l'octopamine des neurones DUM d'insectes et la dopamine des neurones dopaminergiques de la substance noire du cerveau humain, peut laisser envisager qu'un produit actif sur les premiers puisse également l'être sur les seconds.

S'il s'avère que la substance de formule (II) est spécifique aux canaux calciques d'insecte, cette substance pourrait être utilisée dans des insecticides comme indiqué ci-dessus.

Si au contraire, cette substance agit également sur les canaux calciques LVAt de vertébrés, elle pourrait être utilisée pour la fabrication de médicaments activateurs, par exemple, des neurones dopaminergiques, notamment pour soigner la maladie de Parkinson, ainsi que comme médicaments destinés à soigner les troubles cardiaques.

## Revendications

1. Substance neuroactive **caractérisée en ce qu'**elle répond à la formule (0) 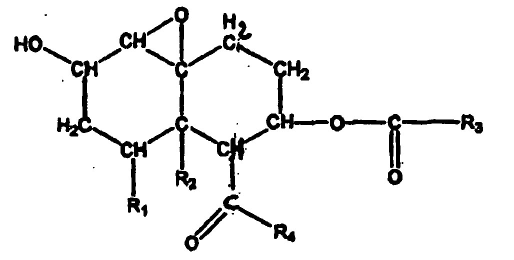 dans laquelle R1, R2, R3 et R4 sont identiques ou différents et sont des radicaux méthyle ou éthyle.

2. Substance neuroactive selon la revendication 1 **caractérisée en ce qu'**elle répond à la formule (I)

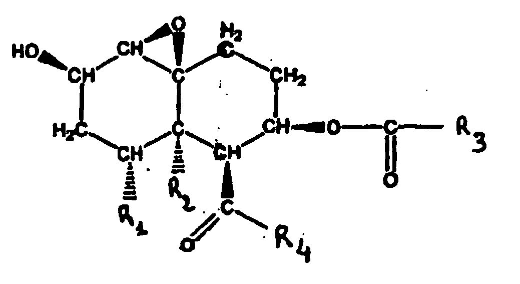

3. Substance neuroactive selon la revendication 2 **caractérisée en ce qu'**elle est constituée par la 6S-acétyt-4R,5R-diméthyl-1R(10S)-époxy-2R-hydroxy-7R-acétoxydécahydronaphtalène répondant à la formule (II)

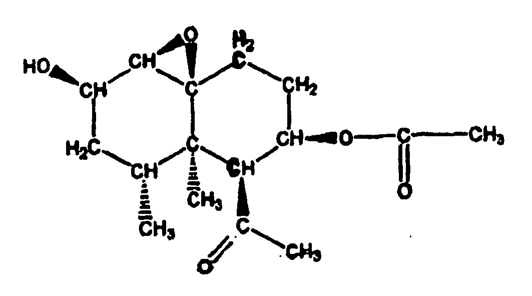

4. Substance selon la revendication 3 **caractérisée en ce que**lle est extraite du cnidaire *Rhytisma fulvum*

5. Substance selon la revendication 1,2 ou 3 **caractérisée en ce qu'**elle est produite par synthèse chimique.

6. Utilisation d'une substance selon l'une quelconque des revendications 1, 2 ou 3 pour la fabrication d'un réactif pharmacologique.

7. Utilisation selon la revendication 6 **caractérisée en ce que** ledit réactif pharmacologique est un activateur sélectif des canaux membranaires calciques transitoires à bas seuil d'activation.

8. Utilisation d'une substance selon l'une quelconque des revendications 1, 2 ou 3 pour la réalisation d'un insecticide.

9. Utilisation selon la revendication 8 **caractérisée en ce que** ladite substance est utilisée en association avec un autre insecticide.

10. Utilisation d'une substance selon la revendication 1, 2 ou 3 pour la fabrication d'un médicament destiné à lutter contre des maladies liées aux troubles de l'excitabilité neuronale.

11. Utilisation selon la revendication 10 pour la fabrication d'un médicament activateur des neurones dopaminergiques.

12. Utilisation selon la revendication 11 pour la fabrication d'un médicament destiné à lutter contre la maladie de Parkinson.

13. Utilisation selon la revendication 10 pour la fabrication d'un médicament destiné à lutter contre les troubles cardiaques.

## Claims

1. Neuroactive substance **characterised in that** it complies with the formula (0) 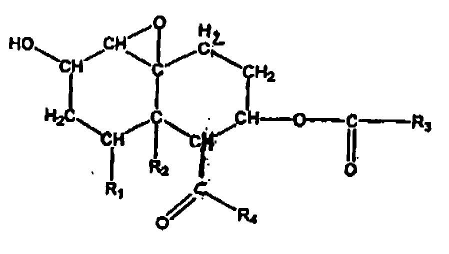 wherein R1, R2, R3 and R4 are identical or different and are methyl or ethyl radicals.

2. Neuroactive substance according to claim 1 **characterised in that** it complies with the formula (I)

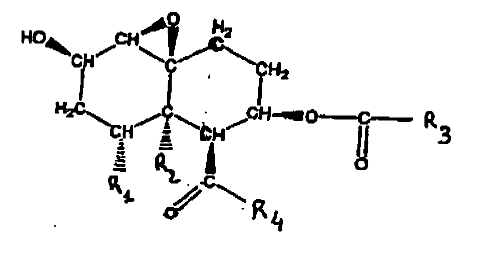

3. Neuroactive substance according to claim 2 **characterised in that** it consists of 6S-acetyl-4R,5R-dimethyl-1R(10S)-epoxy-2R-hydroxy-7R-acetoxydecahydronaphthalene according to formula (II)

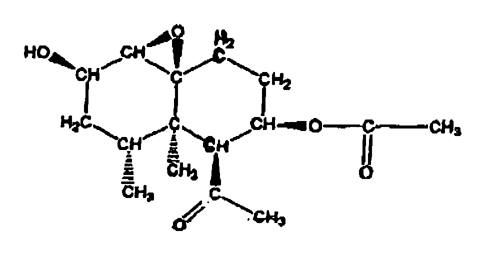

4. Substance according to claim 3 **characterised in that** it is extracted from the Cnidarian *Rhytisma fulvum.*

5. Substance according to claim 1, 2 or 3 **characterised in that** it is produced by chemical synthesis.

6. Use of a substance according to any of claims 1, 2 or 3 to produce a pharmacological reagent.

7. Use according to claim 6 **characterised in that** said pharmacological reagent is a selective transient low voltage activated calcium membrane channel activator.

8. Use of a substance according to any of claims 1, 2 or 3 to produce an insecticide.

9. Use according to claim 8 **characterised in that** said substance is used in association with another insecticide.

10. Use of a substance according to claim 1, 2 or 3 to produce a medicinal product intended to treat diseases associated with neuronal excitability disorders.

11. Use according to claim 10 to produce a dopaminergic neuron activator medicinal product.

12. Use according to claim 11 to produce a medicinal product intended to treat Parkinson's disease.

13. Use according to claim 10 to produce a medicinal product intended to treat the heart disorders.

## Patentansprüche

1. Neuroaktive Substanz, **dadurch gekennzeichnet, dass** sie der Formel (0) 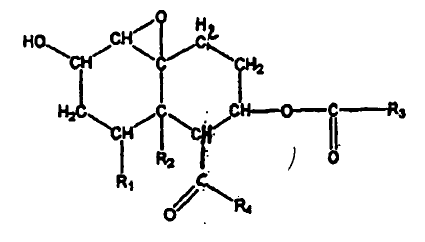 entspricht, in der R1, R2, R3 und R4 identisch oder verschieden und Methylradikale oder Ethylradikale sind.

2. Neuroaktive Substanz nach Anspruch 1, **dadurch gekennzeichnet, dass** sie der Formel (I) entspricht.

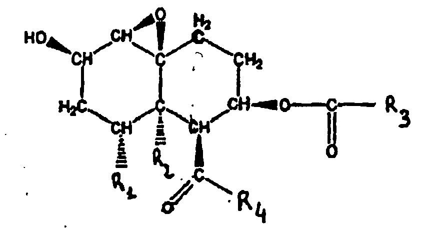

3. Neuroaktive Substanz nach Anspruch 2, **dadurch gekennzeichnet, dass** sie sich aus 6S-Acetyl-4R, 5R-Dimethyl-1 R(10S)-Epoxy-2R-Hydroxy-7R-Acetoxydekahydronaphthalin zusammensetzt entsprechend der Formel (II).

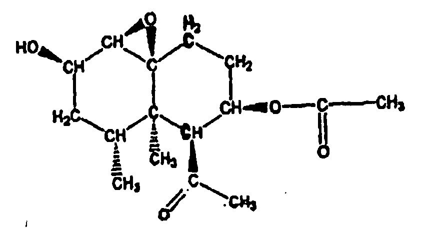

4. Substanz nach Anspruch 3, **dadurch gekennzeichnet, dass** sie aus der Schwefelkoralle *Rhytisma fulvum* extrahiert ist.

5. Substanz nach Anspruch 1, 2 oder 3, **dadurch gekennzeichnet, dass** sie durch chemische Synthese hergestellt ist.

6. Verwendung einer Substanz nach einem der Ansprüche 1, 2 oder 3 zur Herstellung eines pharmakologischen Reagenz.

7. Verwendung nach Anspruch 6, **dadurch gekennzeichnet, dass** das besagte pharmakologische Reagenz selektiver Aktivator transitorischer, niederspannungsaktivierter Kalziumkanäle der Zellmembran ist.

8. Verwendung einer Substanz nach einem der Ansprüche 1, 2 oder 3 zur Herstellung eines Insektizids.

9. Verwendung nach Anspruch 8, **dadurch gekennzeichnet, dass** die besagte Substanz in Verbindung mit einem anderen Insektizid verwendet wird.

10. Verwendung einer Substanz nach Anspruch 1, 2 oder 3 zur Herstellung eines Medikaments gegen Krankheiten, die mit Störungen der neuronalen Erregbarkeit verbunden sind.

11. Verwendung nach Anspruch 10 zur Herstellung eines Medikaments zur Aktivierung Dopamin enthaltender Neuronen.

12. Verwendung nach Anspruch 11 zur Herstellung eines Medikaments gegen die Parkinson-Krankheit.

13. Verwendung nach Anspruch 10 zur Herstellung eines Medikaments gegen Herzerkrankungen.
